# EUROPEAN PATENT APPLICATION

(11) **EP 2 491 932 A2**
(43) Date of publication of application: **29.08.2012**
(21) Application number: 12162497.7
(22) Date of filing: 08.02.2008
(51) Int. Cl.: A61K 31/557, A61P 11/06, A61P 11/00, G01N 33/50

(54) **Use of biomarkers for monitoring the course of treatment of a pulmonary disease upon treatment with treprostinil**

(30) Priority: 09.02.2007 US 900320 P; 25.05.2007 US 940218 P
(62) Divisional of application: 08729432.8
(71) Applicant: United Therapeutics Corporation, Silver Spring, MD 20910 (US)
(72) Inventor: Wade, Michael, Research Triangle Park, 27709 (US); Rich, Stuart, RESEARCH TRIANGLE PARK, 27709 (US); Sullivan, Eugene, OLNEY, 20832 (US); Roscigno, Robert, Research Triangle Park, 27709 (US); Jeffs, Roger, Chapel Hill, 27541 (US)
(74) Representative: Ahner, Francis

(57) **Abstract**

The present invention describes methods for using Treprostinil or its derivative, or a pharmaceutically acceptable salt thereof, for the treatment and/or prevention of interstitial lung disease or asthma, or a condition, such as pulmonary fibrosis, associated with interstitial lung disease or a condition associated with asthma. The invention also relates to kits for treatment and/or prevention of such condition that include an effective amount of Treprostinil or its derivative, or a pharmaceutically acceptable salt thereof.

## Description

### RELATED APPLICATIONS

The present application claims priority to US provisional application no. 60/900,320 filed on February 9, 2007, and US provisional application no. 60/940,218 filed on May 25, 2007, which are incorporated herein by reference in their entirety.

### FIELD

The invention relates to the use of Treprostinil or its derivative, or a pharmaceutically acceptable salt thereof, to treat and/or prevent interstitial lung disease or asthma, or a condition associated with interstitial lung disease or asthma. This invention also relates to kits to be used for this purpose.

### BACKGROUND

### Idiopathic pulmonary fibrosis (IPF)

Five million people are affected by pulmonary fibrosis worldwide, including over 200,000 patients in the United States and the number of deaths from pulmonary fibrosis worldwide is more than 40,000 annually.

Known causes of pulmonary fibrosis include inhaled occupational and environmental pollutants; diseases such as Scleroderma, Rheumatoid Arthritis, Lupus and Sarcoidosis; certain medications with undesirable side effects; therapeutic radiation; genetic/familial conditions. When all known causes are ruled out, the condition is called "idiopathic" pulmonary fibrosis (IPF).

Idiopathic pulmonary fibrosis (IPF) is a progressive disease characterized by alternating areas of normal lung, fibrosis, and interstitial inflammation affecting the peripheral and subpleural parenchyma. Hallmarks of fibrosis include subepithelial myofibroblast/fibroblastic foci and increased deposition of collagen and extracellular matrix. This excess scar tissue causes stiffening of the alveolar walls and a decrease in compliance, which leads to the irreversible loss of total lung capacity and the reduced ability to transport oxygen into the capillaries. Prostanoids, cycolooxygenase-dependant arachidonic acid metabolites, have been implicated in the development of pulmonary fibrosis.

Currently, there is no effective treatment or cure for pulmonary fibrosis. The treatments include administering corticosteroids, alone or in combination with other drugs; oxygen therapy, and lung transplantation. Thus, it is highly desirable to develop a therapy for the treatment of pulmonary fibrosis and other types of interstitial lung disease.

### Asthma

In the United States, over 20 million people have been diagnosed with asthma. Asthma is a complex disorder, characterized by episodic airflow limitation, bronchial hyperresponsiveness, and airway inflammation. The airflow obstruction is typically reversible with administration of bronchodilator drugs; however, with longstanding disease a portion of the obstruction may become irreversible due to a process of airway remodeling. The airway inflammation consists primarily of eosinophils and Th2 lymphocytes.

Prostacyclin (PGI2) may have a role in preventing airway inflammation and remodeling seen in asthma. Hypertrophy/hyperplasia of airway smooth muscle cells contributes to airway narrowing in asthma. PGI2 has an antiproliferative effect on airway smooth muscle (Belvisi, 1998). Mice that are deficient in the prostacyclin receptor (the IP receptor) demonstrate augmented allergen-induced inflammation (Takahashi, 2002; Nagao, 2003) and airway remodeling (Nagao, 2003). Similarly, allergic lung responses (airway eosinophilia, IgE production, airway hyperresponsiveness) are increased in prostaglandin H synthase deficient mice (Gavett, 1999). The Th2 pattern of inflammation is characteristic of asthma. In a mouse ovalbumin model of allergic airway inflammation, PGI2 is produced in the airways and suppresses Th2-mediated allergic inflammation (IL-4, IL-5, IL-13) and airway hyperreactivity (Jaffar, 2002). The prostacyclin analog iloprost, has been shown to have anti-inflammatory effects in a mouse model of asthma. (Idzko, 2007) Iloprost exhibited this effect by interfering with the function of lung myeloid dendritic cells, which are critical antigen-presenting cells of the airways. Iloprost interfered with the maturation and migration of lung dendritic cells to the mediastinal lymph nodes, thereby abolishing the induction of allergen-specific Th2 response in these nodes.

The treatments for asthma include the use of quick release medicines, such as bronchodilators. Long term control medicines for asthma include corticosteroids, inhaled long acting beta-agonists, leukotriene modifiers, cromolyn, nedocromil, and theophyline. There is a need to develop additional therapies for the treatment of asthma.

### SUMMARY

In one embodiment, the present invention is a method for treating or preventing interstitial lung disease or a condition associated with interstitial lung disease, such as pulmonary fibrosis, comprising administration to a subject in need thereof an effective amount of Treprostinil or its derivative, or a pharmaceutically acceptable salt thereof. In one embodiment, the present invention is a method for treating or preventing asthma or a condition associated with asthma, comprising administration to a subject in need thereof an effective amount of Treprostinil or its derivative, or a pharmaceutically acceptable salt thereof. The derivative may be an acid derivative of Treprostinil, a pro-drug of Treprostinil, a sustained release form of Treprostinil, an inhaled form of Treprostinil, an oral form of Treprostinil, a polymorph of Treprostinil or an isomer of Treprostinil. In another embodiment the method of treatment for pulmonary fibrosis is idiopathic pulmonary fibrosis. The fibrosis may be caused by occupational or environmental exposures; pulmonary fibrosis caused by radiation; pulmonary fibrosis caused by connective tissue or collagen diseases; pulmonary fibrosis caused by genetic/familial diseases; pulmonary fibrosis caused by drug side effects; idiopathic pulmonary fibrosis and combinations thereof. Treatment using this invention is also to reduce, eliminate, or prevent pain or other symptom associated with pulmonary fibrosis.

In another embodiment of the invention, the method administers a pharmaceutically acceptable salt of Treprostinil or its derivative, or a pharmaceutically acceptable salt thereof, is administered. The subject of the method may be a mammal or, preferably, a human. Administration may be performed intravenously, by inhalation, or in an orally available form selected from the group consisting of tablets and capsules. In another embodiment, the effective amount is at least 1.0 ng/kg of body weight/min. Alternatively, the effective amount is between 5-500 µg inhaled treprostinil per day.

In another embodiment the current invention is drawn to a method of treating a pulmonary disorder, such as interstitial lung disease, including pulmonary fibrosis, or other conditions, such as asthma, comprising administering a pharmaceutical agent or combination of agents that is known to normalize biomarkers associated with pulmonary disease. In a further embodiment the pharmaceutical agent is treprostinil, the pulmonary disease is IPF, and the biomarkers are MMP-9, Arg-2, VEG-F and PDGF.

In another embodiment, the current invention is a kit for treating or preventing interstitial lung disease or a condition associated with interstitial lung disease, such pulmonary fibrosis, comprising (i) an effective amount of Treprostinil or its derivative, or a pharmaceutically acceptable salt thereof, (ii) one or more pharmaceutically acceptable carriers and/or additives, and (iii) instructions for use in treating or preventing interstitial lung disease. In another embodiment, the current invention is a kit for treating or preventing asthma or a condition associated with asthma, comprising (i) an effective amount of Treprostinil or its derivative, or a pharmaceutically acceptable salt thereof, (ii) one or more pharmaceutically acceptable carriers and/or additives, and (iii) instructions for use in treating or preventing asthma.

In addition, component (i) may be a pharmaceutically acceptable salt of Treprostinil, in a form suitable for intravenous administration, inhalation, or oral administration. The subject treated with the kit may be a mammal or, preferably, a human. The pulmonary fibrosis treated may be is selected from the group consisting of pulmonary fibrosis caused by occupational or environmental exposures; pulmonary fibrosis caused by radiation; pulmonary fibrosis caused by connective tissue or collagen diseases; pulmonary fibrosis caused by genetic/ familial diseases; pulmonary fibrosis caused by drug side effects; idiopathic pulmonary fibrosis and combinations thereof.

### DETAILED DESCRIPTION

The current invention relates to therapies that-enhance blood flow by increasing blood flow though smaller vessels and capillaries, and are effective to treat and prevent interstitial lung disease or conditions associated with interstitial lung disease, such as pulmonary fibrosis.

The current invention also relates to therapies that-are effective to treat and prevent asthma, or conditions associated with asthma.

Prostacyclin is a small molecule that has been previously shown to cause dilation of large blood vessels, relaxation of smooth muscle, inhibition of smooth muscle proliferation, as well as inhibition of platelet aggregation, which is involved in the blood clotting process. Similar actions by Treprostinil at the microvascular level and on capillaries near the skin are believed to help enhance cutaneous blood flow and heal and/or prevent ischemia lesions or ulcers associated with scleroderma, Buerger's disease, Raynaud's disease, Raynaud's phenomenon, and other conditions.

The present invention relates to methods for treating and/or preventing interstitial lung disease or asthma, or a condition associated with interstitial lung disease or asthma, comprising administering to a subject in need thereof an effective amount of Treprostinil and/or a derivative thereof and/or a pharmaceutically acceptable salt thereof. Suitable derivatives include acid derivatives, pro-drugs, sustained release forms, inhaled forms and oral forms of Treprostinil, including those disclosed in U.S. Patents Nos. 6,521,212 and 6,756,033 to Cloutier *et. al.* and US patent application publications Nos. 20050085540 and 20050282901 to Phares *et. al.*

Unless otherwise specified, the term "a" or "an" used herein shall mean "one or more."

As used herein, the phrase "instructions for use" shall mean any FDA-mandated labeling, instructions, or package inserts that relate to the administration of Treprostinil or its derivatives, or pharmaceutically acceptable salts thereof, for the purpose of treating or preventing interstitial lung disease or asthma, or conditions associated with interstitial lung disease or asthma. For example, instructions for use may include, but are not limited to, indications for asthma, or conditions associated interstitial lung disease, such as pulmonary fibrosis, or conditions associated with asthma, identification of specific symptoms associated with such conditions that can be ameliorated by Treprostinil, and recommended dosage amounts for subjects suffering from interstitial lung disease or asthma.

The term "acid derivative" is used herein to describe C₁-C₄ alkyl esters and amides, including amides wherein the nitrogen is optionally substituted by one or two C₁-C₄ alkyl groups.

Many acute and chronic lung disorders with variable degrees of inflammation and fibrosis are collectively referred to as interstitial lung diseases (ILDs). Because of the stiff fibrosis of the lung, pulmonary arterial hypertension (PAH) is often a late complication of some forms of ILD.

Pulmonary hypertension includes multiple diseases such as pulmonary arterial hypertension (PAH) and pulmonary venous hypertension.

The term "pulmonary fibrosis" is a condition in which the tissue-of the lungs has become thick and scarred. The condition is well established in the medical community-and is associated with shortness of breath, fatigue, weakness, chronic dry, hacking cough, loss of appetite, and discomfort in the chest. Over time the scarring in the lung becomes replaced with fibrotic tissue and the lung tissue becomes thicker. This thickening causes a loss in the lung's ability to transfer oxygen to the blood. This condition is distinct from other pulmonary conditions-such as pulmonary hypertension.

The term "asthma" is a condition in which the inside of the airways which carry air to the lungs become inflamed. The condition is well established in the medical community. This inflammation causes narrowing of the airways and obstruction to air flow. This condition is distinct from other pulmonary conditions.

The invention also includes bioprecursors or "pro-drugs" of Treprostinil, that is, compounds which are converted *in vivo* to Treprostinil or its pharmaceutically active derivatives thereof.

Further aspects of the present invention are concerned with the use of Treprostinil or its derivatives, or pharmaceutically acceptable salts thereof, in the manufacture of a medicament for the treatment or prevention of interstitial lung disease or asthma, or a condition associated with interstitial lung disease or asthma.

The present invention also encompasses methods of using Treprostinil or its derivatives, or pharmaceutically acceptable salts thereof. In one embodiment, a method uses Treprostinil sodium, currently marketed under the trade name of REMODULIN®. The FDA has approved Treprostinil sodium for the treatment pulmonary arterial hypertension by injection of dose concentrations of 1.0 mg/mL, 2.5 mg/mL, 5.0 mg/mL and 10.0 mg/mL. The chemical structure formula for Treprostinil sodium is:

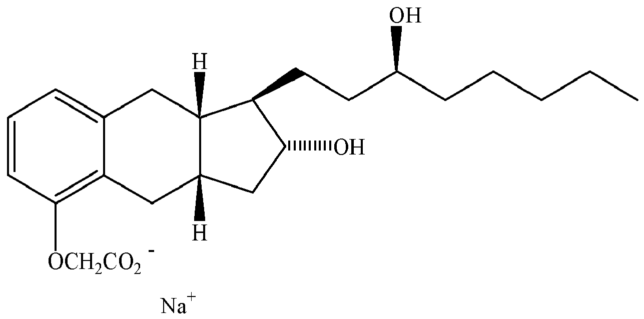

Treprostinil sodium is sometimes designated by the chemical names: (a) [(1*R*,2*R*,3a*S*,9a*S*)-2,3,3a,4,9,9a-hexahydro-2-hydroxy-1-[(3*S*)-3-hydroxyoctyl]-1*H*-benz [*f*]inden-5-yl]oxy] acetic acid; or (b) 9-deoxy-2',9-α-methano-3-oxa-4,5,6-trinor-3,7-(1',3'-interphenylene)-13,14-dihydro-pr ostaglandin F₁. Treprostinil sodium is also known as: UT-15; LRX-15; 15AU81; UNIPROST™; BW A15AU; and U-62,840. The molecular weight of Treprostinil sodium is 390.52, and its empirical formula is C₂₃H₃₄O₅.

The present invention extends to methods of using physiologically acceptable salts of Treprostinil, as well as non-physiologically acceptable salts of Treprostinil that may be used in the preparation of the pharmacologically active compounds of the invention.

Physiologically acceptable salts of Treprostinil include salts derived from bases. Base salts include ammonium salts (such as quaternary ammonium salts), alkali metal salts such as those of sodium and potassium, alkaline earth metal salts such as those of calcium and magnesium, salts with organic bases such as dicyclohexylamine and N-methyl-D-glucamine, and salts with amino acids such as arginine and lysine.

Quaternary ammonium salts can be formed, for example, by reaction with lower alkyl halides, such as methyl, ethyl, propyl, and butyl chlorides, bromides, and iodides, with dialkyl sulphates, with long chain halides, such as decyl, lauryl, myristyl, and stearyl chlorides, bromides, and iodides, and with aralkyl halides, such as benzyl and phenethyl bromides.

The amount of Treprostinil or its derivative, or a pharmaceutically acceptable salt thereof, that is required in a medication or diagnostic aid according to the invention to achieve the desired effect will depend on a number of factors, such as the specific application, the nature of the particular compound used, the mode of administration, the concentration of the compound used, and the weight and condition of the patient. A daily dose per patient for treatment or prevention of interstitial lung disease or asthma, or conditions associated with interstitial lung disease or asthma may be in the range 25 µg to 250 mg; 0.5 µg to 2.5 mg, or 7 µg to 285 µg, per day per kilogram bodyweight. For example, an intravenous dose in the range 0.5 µg to 1.5 mg per kilogram bodyweight per day may conveniently be administered as an infusion of from 0.5 ng to 1.0 µg per kilogram bodyweight per minute. One possible dosage is 2.5 ng/kg/min, increased over 12 weeks by an amount of 2.50 ng/kg/min each week, until a target dose, such as 15 ng/kg/min, is reached. Infusion fluids suitable for this purpose contain, for example, from 10 ng to 1 µg per milliliter. Ampoules for injection contain, for example, from 0.1 µg to 1.0 mg and orally administrable unit dose formulations, such as tablets or capsules, contain, for example, from 0.1 to 100 mg, typically from 1 to 50 mg. For diagnostic purposes, a single unit dose formulation may be administered. In the case of physiologically acceptable salts, the weights indicated above refer to the weight of the active compound ion, that is, the ion derived from Treprostinil.

In the manufacture of a medicament or diagnostic aid according to the invention, hereinafter referred to as a "formulation," Treprostinil and/or its derivatives, and/or pharmaceutically acceptable salts thereof, may be admixed with, inter alia, an acceptable carrier. The carrier must, of course, be acceptable in the sense of being compatible with any other ingredients in the formulation and must not be deleterious to the subject. The carrier may be a solid or a liquid, or both, and is preferably formulated with the compound as a unit-dose formulation, for example, a tablet, which may contain from 0.05% to 95% by weight of the active compound. One or more of Treprostinil or its derivatives, or pharmaceutically acceptable salts thereof, may be incorporated in the formulations of the invention, which may be prepared by any of the well known pharmaceutical techniques for admixing the components.

The formulations of the invention include those suitable for parenteral (e.g., subcutaneous, intramuscular, intradermal, or intravenous), oral, inhalation (in solid and liquid forms), rectal, topical, buccal (e.g., sub-lingual) and transdermal administration, although the most suitable route in any given case may depend on the nature and severity of the condition being treated and on the nature of the particular form of Treprostinil, its derivative, or a pharmaceutically acceptable salt thereof.

Formulations of the present invention suitable for parenteral administration conveniently comprise sterile aqueous preparations of Treprostinil or its derivative, or a pharmaceutically acceptable salt thereof, where the preparations may be isotonic with the blood of the intended recipient. These preparations may be administered by means of subcutaneous injection, although administration may also be effected intravenously or by means of intramuscular or intradermal injection. Such preparations may conveniently be prepared by admixing the compound with water or a glycine or citrate buffer and rendering the resulting solution sterile and isotonic with the blood. Injectable formulations according to the invention may contain from 0.1 to 5% w/v of active compound and may be administered at a rate of 0.1 ml/min/kg. Alternatively, the invention may be administered at a rate of 0.625 to 50 ng/kg/min. Alternatively, the invention may be administered at a rate of 10 to 15 ng/kg/min.

Formulations suitable for oral administration may be presented in discrete units, such as capsules, cachets, lozenges, or tablets, each containing a predetermined amount of Treprostinil or its derivative, or a pharmaceutically acceptable salt thereof; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients).

In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations suitable for buccal (sub-lingual) administration include lozenges comprising Treprostinil or its derivative, or a pharmaceutically acceptable salt thereof, in a flavored base, usually sucrose and acacia or tragacanth; and pastilles comprising the compound in an inert base such as gelatin and glycerin or sucrose and acacia.

Formulations suitable for rectal administration are preferably presented as unit dose suppositories. These may be prepared by admixing Treprostinil or its derivative, or a pharmaceutically acceptable salt thereof, with one or more conventional solid carriers, for example, cocoa butter, and then shaping the resulting mixture.

Formulations suitable for topical application to the skin preferably take the form of an ointment, cream, lotion, paste, gel, spray, aerosol, or oil. Carriers which may be used include vaseline, lanoline, polyethylene glycols, alcohols, and combinations of two or more thereof. The active compound is generally present at a concentration of from 0.1 to 15% w/w, for example, from 0.5 to 2% w/w. Formulations for transdermal administration may be delivered by iontophoresis (see, for example, Pharmaceutical Research, 3(6): 318 (1986)) and typically take the form of an optionally buffered aqueous solution of Treprostinil or its derivative or salt or thereof. Suitable formulations comprise citrate or bis/tris buffer (pH 6) or ethanol/water and contain from 0.1 to 0.2M active ingredient.

The compounds of the present invention are conveniently prepared by methods the same as or analogous to those described in U.S. Pat. No. 4,306,075, U.S. Pat. No. 6,528,688 and U.S. Pat. No. 6,441,245.

In certain kit embodiments, the Treprostinil or its derivative, or a pharmaceutically acceptable salt thereof, is in a form suitable for subcutaneous administration, continuous subcutaneous infusion, intravenously administration or inhalation. In other kit embodiments, the Treprostinil or its derivative, or a pharmaceutically acceptable salt thereof, is in an orally available form selected from the group consisting of tablets and capsules. In another kit embodiment, the effective amount of Treprostinil or its derivative, or a pharmaceutically acceptable salt thereof, is at least 1.0 ng/kg of body weight/min.

Effect of treprostinil on pulmonary fibrosis can be tested using an animal model of pulmonary fibrosis such as bleomycin and V2O5 models of pulmonary fibrosis described in Bonner JC, Rice AB, Ingram JL, Moomaw CR, Nyska A, Bradbury A, Sessoms AR, Chulada PC, Morgan DL, Zeldin DC, and Langenbach R. Susceptibility of cyclooxygenase-2-deficient mice to pulmonary fibrogenesis. Am J Pathol 161: 459-470, 2002; 23; and Keerthisingam CB, Jenkins RG, Harrison NK, Hernandez-Rodriguez NA, Booth H, Laurent GJ, Hart SL, Foster ML, and McAnulty RJ. Cyclooxygenase-2 deficiency results in a loss of the anti-proliferative response to transforming growth 31 factor-beta in human fibrotic lung fibroblasts and promotes bleomycin-induced pulmonary fibrosis in mice. Am J Pathol 158: 1411-1422, 2001, incorporated herein by reference in their entirety.

Formulations of the current invention may also be employed to normalize biomarkers associated with pulmonary disease. Pulmonary disease and affected cells or tissue are associated with varied concentrations of proteins and cellular compounds. These compounds provide biomarkers to assess the severity and course of disease. For example, matrix metalloproteinase 9 (MMP-9), angiopoetin-2 (Ang-2), vascular endothelium-derived growth factor (VEG-F), and platelet derived growth factor (PDGF) are associated with lung disease and can be used in this invention to monitor the course of treatment with treprostinil or other pharmaceutical agent.

Effect of treprostinil on asthma can be tested using an animal model of asthma such as a murine model of chronic human asthma. See Kumar RK and Foster PS., Immunol Cell Biol. 2001 Apr; 79(2): 141-4.

The disclosure of all publications cited above are expressly incorporated herein by reference in their entireties to the same extent as if each were incorporated by reference individually.

The examples described herein are illustrative of the present invention and are not intended to be limitations thereon. Different embodiments of the present invention have been described according to the present invention. Many modifications and variations may be made to the techniques described and illustrated herein without departing from the spirit and scope of the invention. Accordingly, it should be understood that the examples are illustrative only and are not limiting upon the scope of the invention.

### EXAMPLES

### Example 1

### Bleomycin Induced Fibrosis

Bleomycin-induced fibrosis has been used extensively to model aspects of the pathogenesis of pulmonary fibrosis. See, for example, Smith, et al., J. Immunol. 153:4704 (1994).

Experimental and control mice are treated with bleomycin and analyzed to verify development of pulmonary fibrosis like symptoms. Delivery of Treprostinil is at 150 ng/kg/min. Typical experimental mice are about 20g, allowing for a delivery of 180 ng/hr subcutaneously, or 1.8x10-4 mg/hr. Treprostinil concentration in solution is 7.2x10-4 ug/ul.

18 total mice are studied, 9 receiving Treprostinil and 9 receiving placebo. 3 mice from each group are analyzed after days 7, 14, and 21 to compare the affect of Treprostinil on tissue. After the period of days described above, mice are analyzed in order to determine the effect on treprostinil treated and control mice. The effect of non-bleomycin treated mice is compared with both the bleomycin treated mice without treprostinil and with treprostinil to show the effectiveness of treprostinil administration on the course of pulmonary fibrosis.

### Example 2

### Ovalbumin Model for Lung Disease

Ovalbumin sensitive mice have been used extensively to model aspects of the pathogenesis of lung disease including asthma.

Experimental and control mice are treated with ovalbumin and analyzed to verify development of asthma like findings. Typical experimental mice are about 20g. Treprostinil may be delivered in various dosage forms well known to one of skill in the art and totaling about 4.32 µg/day to the mice.

18 total mice are studied, 9 receiving Treprostinil and 9 receiving placebo. 3 mice from each group are analyzed after days 7, 14, and 21 to compare the affect of Treprostinil on tissue. After the period of days described above, mice are analyzed in order to determine the effect on treprostinil treated and control mice. The effect of non-ovalbumin treated mice is compared with both the ovalbumin treated mice without treprostinil and with treprostinil to show the effectiveness of treprostinil administration on the course of asthma.

### Example 3

The mouse model described in Belperio et al. is used to assess treatment of pulmonary fibrosis with Treprostinil. See Belperio, J. et al, Critical role for the chemokine MCP-1/CCR2 in the pathogenesis of bronchioligits obliterans syndrome, Journal of clinical investigation 108: 547, 2001. This model looks at tracheas from babl/c mice transplanted onto the backs of c57BL/6 mice, where the tracheas are histoathologically scored for pathological processes.

Pumps can be used to deliver Treprostinil and placebo to mice. The Alzet osmotic pump 2004 hold 200ul and has a flow rate of 0.25 ul/hr. Other pumps may be utilized as appropriate. Delivery of Treprostinil is at 150 ng/kg/min. Typical experimental mice are about 20g, allowing for a delivery of 180 ng/hr subcutaneously, or 1.8x10-4 mg/hr. Treprostinil concentration in solution is 7.2x10-4 ug/ul.

4 balb/c tracheas per C57BL/6 backs.

18 total mice are studied, 9 receiving Treprostinil and 9 receiving placebo. 3 mice from each group are analyzed after days 7, 14, and 21 to compare the affect of Treprostinil on tissue. After the period of days described above, mice are analyzed in order to determine the effect on treprostinil treated and control mice. All samples are analyzed for histopath and murine BOS scoring.

### Example 4

The effect of treprostinil (in the form ofRemodulin or inhaled treprostinil) on patients is analyzed using the 6-minute walk test with Borg dyspnea score, a standard assessment of exercise capacity and breathlessness in patients with lung disease. See Guyatt, G. Sullivan, M. et al. (Canada Medical Assoc. J. Vol. 132, 1985). The Six-Minute Walk corresponds closely to the demands of everyday activity and is a safe and simple measurement of functional exercise capacity for clinical trials in patients.

Remodulin is administered to patients intravenously or subcutaneously in the range of 2.5 to 80 ng/kg/min. Inhaled treprostinil is administered to patients orally in the range of 5-60 µg 4 times daily. Two groups of subjects, one subject group receiving drug and one control group receiving placebo are studied. Subjects receive placebo or drug for the entire 12 week study and are tested periodically, for example every two weeks, using the six minute walk test.

### General Procedures

The area used for the Six-Minute Walk test should be pre-measured at a minimum of 108 feet (33 meters) in length and at least 6 to 10 feet (2 to 3 meters) in width. The length should be marked with half-yard (0.5 meter) gradations. The area should be well ventilated with air temperature controlled at 20 to 23 °C (68 to 76 °F).

The tester may be at the starting end of the corridor or at the midpoint of the corridor with a stop-watch. Intermittent rest periods are allowed if the patient can no longer continue. If the patient needs to rest briefly, he/she may stand or sit and then begin again when he/she is sufficiently rested but the clock will continue to run. At the end of six minutes, the tester will call "stop" while simultaneously stopping the watch and then measure the distance walked. The Borg Dyspnea Rating is then administered.

### Six Minute Walk Exercise Test

Instructions to the Patient

Patients is instructed that the preceding meal should be light. Patients should be told to wear comfortable clothing and sneakers or comfortable walking shoes. The person administering the test uses the following dialogue with the patient:

"The purpose of this test is to find out how far you can walk in six minutes. You will start from this point and follow the hallway to the marker (e.g. chair) at the end, turn around and walk back. When you arrive back at the starting point you will go back and forth again. You will go back and forth as many times as you can in the six-minute period. You may stop and rest if you need to. Just remain where you are until you can go on again. However, the most important thing about the test is that you cover as much ground as you possibly can during the six minutes. I will tell you the time, and I will let you know when the six minutes are up. When I say STOP, please stand right where you are."

After these instructions are given to the patient, the person administering the test asks: "Do you have any questions about the test?" "Please explain to me what you are going to do."

The person administering the test then starts the test by saying the following to the patient: "Are you ready?" "Start when I say "GO."

The person administering the test tells the patient the time at 2 and 4 minutes by saying: "You have completed 2 minutes." And then by saying: "You have completed 4 minutes."

No other instruction or encouragement are given during the test. Eye contact with the patient should be avoided during the test. Following the walk, the person administering the test obtains a rating of dyspnea using the Borg Scale. The person uses the following dialogue:

### Borg Dyspnea Score

"I would like to use the following scale to indicate the maximal shortness of breath you had during the walk test (indicate the Borg Scale). If there was no shortness of breath at all you would point to 0; if the shortness of breath was not very great you would choose from 0.5 to 2; if you were somewhat more short of breath you would select 3; and if the breathing was getting very difficult, you would choose 4 to 9, depending on just how hard it was; 10 represent the greatest shortness of breath you have ever experienced in your life, and if you feel more short of breath than you have ever been in you life before, choose a number greater that 10 that represents how short of breath you feel. If one of the numbers does not exactly represent how short of breath you are, then you can choose a fraction between. For example, if you had shortness of breath somewhere between 4 and 5, you could choose 4½.

### Example 5

This Example shows the effect of treprostinil on biomarkers associated with pulmonary disease. In addition, treatment with treprostinil is shown to have positive outcome in the six minute walk test described above.

44 patients with PAH received study drug (Remodulin or placebo). 30 patients received Remodulin and 14 received placebo. A 12-week trial was conducted. The mean dose of Remodulin received was 72.5 ng/kg/min at Week 12 (compared to 80.0 ng/kg/min placebo-equivalent). Remodulin produced an 93.0 meter median improvement in the six-minute walk compared to placebo, Hodges-Lehmann estimate, with a 95% CI of 7.0 to 187.0 (p = 0.0077) from nonparametric ANCOVA. Remodulin also significantly improved combined ranking of 6MW/Borg Score Index (p = 0.0023), Borg Dyspnea score (2.0 median improvement over placebo, p =0.23), and other confirmatory efficacy endpoints.

These results indicate that for patients with pulmonary hypertension, Remodulin has a positive impact on the inflammatory processes important in IPF. This suggests that Remodulin can treat both the symptoms of IPF, such as diminished lung function and exercise capacity, and ameliorate the pulmonary disease processes in both pulmonary hypertension and IPF.

Although the mechanism may differ in the context of asthma, the beneficial effects of treprostinil on lung pathology will alter the biomarkers associated with asthma.

Baseline patients with PAH appeared to have higher-than-normal levels of MMP-9, Ang-2, VEG-F, and PDGF (matrix metalloproteinase 9, angiopoetin-2, vascular endothelium-derived growth factor, and platelet-derived growth factor, respectively). Remodulin treatment for 12 weeks significantly decreased serum Ang-2 and VEG-F. There was also a strong trend toward decreased PDGF.

Further, there was a statistically significant correlation between the degree of decrease in Ang-2 and the degree of improvement in the six-minute walk test. In addition, there was a statistically significant correlation between reductions in MMP-9 and clinical improvement observed in the six-minute walk distance, although the degree of change in MMP-9 alone was not significant.

### Example 6

The following study shows the effect of intravenous treprostinil in patients with idiopathic pulmonary fibrosis and pulmonary hypertension. The following measurements are taken in subjects: 1) change from baseline to week 12 in 6-minute walk distance (6MWD), 2) change from baseline to week 12 in hemodynamic parameters (RHC) at rest, and 3) New York Heart Association (NYHA) class from baseline to week 12. Other measurements provided in this study are: 1) change from baseline 02 desaturation and quantity of desaturation measures during 6MWD at week 6 and 12, 2) change from baseline forced vital capacity (FVC) and Diffusing Capacity (DLCO) at weeks 6 and 12, 3) change from baseline in dyspnea using Borg scale at weeks 6 and 12, 4) change from baseline to week 12 in hemodynamics (RHC) at exercise using cycle geometry, and 5) analysis of "signaling cascades" will attempt to determine differences between cytokine/chemokine/growth factor and down stream signaling cascades between IPF and IPF/PAH. These cytokine/chemokine/growth factor and down stream signaling cascade profiles may predict which group will have a more aggressive decline.

The initial dose is 1.25 ng/kg/minute treprostinil and the infusion rate is titrated up as the patient tolerates by increase dose 1-2ng/kg/min three time per week until a maximum dose of 40 ng/kg/min is reached or the subject has dose-limiting adverse effects (including but not limited to: hypotension, infusion site reaction, infusion site pain, headaches, diarrhea, jaw pain, vomiting, or flushing).

As part of standard of care the following procedures are performed on subjects: right heart catheterization, transthoracic echocardiogram, 6 minute walk, full pulmonary function tests, HRCT chest and a battery of blood tests (BNP, DDimer, CRP, Troponin I, and liver function testing).

At the time of the initial right heart catheterization, blood is collected from the cordis port for cytokine/chemokine/growth factor and down stream signaling cascade profile analysis. Also standard of care blood work is done on the day of catheterization testing for BNP, C reactive protein, D-Dimer, Troponin-I and liver function testing. Study blood work includes 4cc of blood into each of four tubes including dark green, purple, red and yellow tops. Blood work (both standard of care and study blood) is repeated on a scheduled basis for all patients enrolled into the study.

Borg Dyspnea Score is done at the initial 6 MW and with subsequent 6MW done per scheduled (listed below) thereafter. NYHA functional class is determined at the initiation into the study and as scheduled thereafter.

### DATA TIME POINTS FLOW DIAGRAM

| Initial | 6weeks | 12weeks | 6months | 1 Year |
|---|---|---|---|---|
| R heart cath | | | | R heart cath |
| 6MW | 6MW | 6MW | 6MW | 6MW |
| | | | | |
| TTE | | | | TTE |
| Dyspnea Score | Dyspnea Score | Dyspnea Score | Dyspnea Score | Dyspnea Score |
| Blood work (lab) | | Blood work (lab) | | Blood work (lab) |
| QOL index | | | | QOL index |
| Spirometer DLCO (lab) | | Spirometer DLCO (lab) | | Spirometer DLCO (lab) |
| NYHA/ WHO class | | | | NYHA/ WHO class |
| HRCT chest | | | | HRCT chest |

Subjects receiving Treprostinil will show improvement in the studied criteria indicating the positive effect of treprostinil treatment in patients with idiopathic pulmonary fibrosis and pulmonary hypertension.

### References

Belvisi MG, Saunders M, Yacoub M, and Mitchell JA. Expression of cyclooxygenase-2 in human airway smooth muscle is associated with profound reductions in cell growth. Br J Pharmacol, 1998. 125:1102-1108.

Gavett SH, Madison SL, Chulada PC, et. al. Allergic lung responses are increased in prostaglandin H synthase-deficient mice. J Clin Invest, 1999; 104:721-732.

Idzko M, Hammad H, van Nimwegen M, et al. Inhaled iloprost suppresses the cardinal features of asthma via inhibition of airway dendritic cell function. J Clin Invest, 2007. 117: 464-472.

Jaffar Z, Wan K-S, and Roberts K. A key role for prostaglandin I2 in limiting lung mucosal Th2, but not Th1, responses to inhaled allergen. J Immunol, 2002; 169:5997-6004.

Nagao K, tanaka H, Komai M, et. al. Role of prostaglandin I2 in airway remodeling induced by repeated allergen challenge in mice. Am J Respir Cell Mol Biol, 2003; 29: 314-320.

Takahashi Y, Tokuoka S, Masuda T, et. Al. Augmentation of allergic inflammation in prostanoid IP receptor deficient mice. Br J Pharmacol, 2002; 137:315-322.

## Claims

1. Use of at least one biomarker selected from the group consisting of MMP-9, ARG-2, Veg-F and PDGF for monitoring the course of treatment of a pulmonary disease upon treatment with a pharmaceutical agent.

2. The use of claim 1, wherein the pharmaceutical agent comprises treprostinil or a pharmaceutically acceptable salt thereof.

3. The use of claim 1, wherein the pulmonary disease is pulmonary arterial hypertension.

4. The use of claim 1, wherein the pulmonary disease is idiopathic pulmonary fibrosis.

5. The use of claim 1, wherein said treatment normalizes the level of the at least one biomarker.

6. Use of treprostinil for treatment of a pulmonary disease or a pharmaceutically acceptable salt thereof, wherein said treatment comprises monitoring at least one biomarker of the pulmonary disease.

7. The use of claim 6, wherein the at least one biomarker is selected from the group consisting of MMP-9, ARG-2, Veg-F and PDGF.

8. The use of claim 6, wherein the pulmonary disease is pulmonary arterial hypertension.

9. The use of claim 6, wherein the pulmonary disease is idiopathic pulmonary fibrosis.

10. The use of claim 6, wherein said treatment normalizes the level of the at least one biomarker.

11. Use of a pharmaceutically effective amount of treprostinil or a pharmaceutically acceptable salt thereof in an parenteral medicament for treating pulmonary hypertension, which is a complication of interstitial lung disease.

12. The use of claim 11, wherein the medicament is for intravenous administration.

13. The use of claim 11, wherein the medicament is for subcutaneous infusion, including continuous subcutaneous infusion.

14. The use of any one of claims 11 in a mammal, such as a human being.

15. The use of any one of claims 11, wherein administration of the medicament results in an improvement of a six minute walk test.
